(19) Europäisches Patentamt / European Patent Office / Office européen des brevets

(11) EP 2 557 081 A1

(12) **EUROPEAN PATENT APPLICATION**
published in accordance with Art. 153(4) EPC

(43) Date of publication:
**13.02.2013 Bulletin 2013/07**

(21) Application number: **11756256.1**

(22) Date of filing: **14.03.2011**

(51) Int Cl.:
***C07D 495/04*** $^{(2006.01)}$ ***H01L 29/786*** $^{(2006.01)}$
***H01L 51/05*** $^{(2006.01)}$ ***H01L 51/30*** $^{(2006.01)}$

(86) International application number:
**PCT/JP2011/055963**

(87) International publication number:
**WO 2011/115071 (22.09.2011 Gazette 2011/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2010 JP 2010058527**

(71) Applicants:
- **Hiroshima University**
  **Hiroshima 739-8511 (JP)**
- **Kuraray Co., Ltd.**
  **Okayama 710-0801 (JP)**

(72) Inventors:
- **OHSHITA, Joji**
  **Higashihiroshima-shi**
  **Hiroshima 739-8527 (JP)**
- **HARIMA, Yutaka**
  **Higashihiroshima-shi**
  **Hiroshima 739-8527 (JP)**
- **SUGIOKA, Takashi**
  **Kurashiki-shi**
  **Okayama 710-0801 (JP)**
- **KANEHIRA, Koichi**
  **Kurashiki-shi**
  **Okayama 710-0801 (JP)**

(74) Representative: **Gallagher, Kirk James**
**D Young & Co LLP**
**120 Holborn**
**London EC1N 2DY (GB)**

# (54) THIENOPYRIDINE DERIVATIVE, METHOD FOR PRODUCING SAME AND ORGANIC SEMICONDUCTOR DEVICE USING SAME

(57) Provided are a novel thienopyridine derivative represented by the following general formula (1), useful as an organic semiconductor device, especially an organic thin film transistor element, and a method for the production thereof:

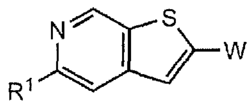

(1)

(wherein $R^1$ is a hydrogen atom, an alkyl group that may have a substituent, an alkenyl group that may have a substituent, an alkynyl group that may have a substituent, an alkoxy group that may have a substituent, an aryl group that may have a substituent, a heteroaromatic ring group that may have a substituent, an alkylthio group that may have a substituent, an arylthio group that may have a substituent, an ester group that may have a substituent, or a substituent represented by $-SO_2R^2$ (wherein $R^2$ is a hydrocarbon group having 1 to 20 carbon atoms that may have a substituent), W is at least one member selected from the group consisting of aryl groups that may have a substituent and heteroaromatic ring groups that may have a substituent.)

EP 2 557 081 A1

## Description

### TECHNICAL FIELD

The present invention relates to a novel compound having a thienopyridine skeleton, a method for producing the same, and an organic semiconductor device using the same.

### BACKGROUND ART

In recent years, nanometric π-conjugated molecules have attracted attention as various functional materials, such as electrically conductive materials, photoelectric conversion materials, electroluminescent materials, non-linear optical materials, and field effect transistor materials, especially as organic semiconductor materials. Generally, in the case of an organic semiconductor material composed of a low-molecular compound, an organic semiconductor layer can be formed by a process involving vapor deposition performed at a comparatively low temperature. Moreover, irrespective of whether the organic semiconductor material is composed of a low-molecular compound or a high-molecular compound, an organic semiconductor layer can be formed by a solution process such as a printing process like an inkjet method. Therefore, as compared with the case of preparation using an inorganic semiconductor material, a greater cost reduction can be achieved by the preparation of an organic semiconductor element using an organic semiconductor material.

On the other hand, organic semiconductor materials have a drawback that they are lower in carrier mobility than inorganic semiconductor materials, such as silicon. As one measure to solve this drawback, there have been proposed organic semiconductor materials, such as thiophenes, e.g., oligothiophene and polythiophene, acenes, e.g., pentacene and rubrene, and fused ring compounds, e.g., benzothiophene and thienothiophene. Among these, low-molecular materials typified by oligothiophene are one of the organic semiconductor materials that are being studied most because they have an advantage that they are superior in packing property due to their unique molecular size, they can be relatively easily synthesized, they are stable to heat and light, and they can be structurally modified precisely (Non-patent Document 1). However, since organic semiconductor materials are not necessarily sufficient in properties required of organic semiconductor elements typified by carrier mobility and so on, provision of novel organic semiconductor materials has been desired for the purpose of further improvement.

### PRIOR ART DOCUMENT

### NON-PATENT DOCUMENT

Non-patent Document 1: T. Otsubo et al., Synthesis, Optical, and Conductive Properties of Long Oligothiophenes and Their Utilization as Molecular Wires, Bull. Chem. Soc. Jpn. 2001, Vol.74, No.10,p.1789-1801

### SUMMARY OF THE INVENTION

### PROBLEM TO BE SOLVED BY THE INVENTION

The present invention has been made in order to solve the above-mentioned problems and an object thereof is to provide a novel thienopyridine derivative useful as an organic semiconductor device, especially an organic thin film transistor element, and to provide a method for the production thereof.

### MEANS FOR SOLVING THE PROBLEMS

The aforementioned problem is solved by providing a thienopyridine derivative represented by the following general formula (1):

[Chem. 1]

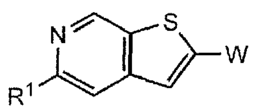

(1)

wherein $R^1$ is a hydrogen atom, an alkyl group that may have a substituent, an alkenyl group that may have a substituent, an alkynyl group that may have a substituent, an alkoxy group that may have a substituent, an aryl group that may have a substituent, a heteroaromatic ring group that may have a substituent, an alkylthio group that may have a substituent, an arylthio group that may have a substituent, an ester group that may have a substituent, or a substituent represented by $-SO_2R^2$ (wherein $R^2$ is a hydrocarbon group having 1 to 20 carbon atoms that may have a substituent), W is at least one member selected from the group consisting of aryl groups that may have a substituent and heteroaromatic ring groups that may have a substituent.

Preferably in the above, W is a heteroaromatic ring group represented by the following general formula (2):

[Chem. 2]

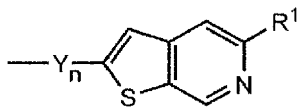

(2)

wherein $R^1$ is the same as defined above for the general formula (1), Y is at least one member selected from the group consisting of arylene groups and divalent heteroaromatic ring groups, and n is an integer of not less than 0.

It is one preferred embodiment of the present invention to provide a method for producing the thienopyridine derivative, wherein the method comprises subjecting a compound represented by the following general formula (3):

[Chem. 3]

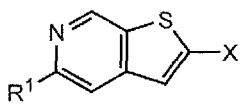

(3)

wherein $R^1$ is the same as defined above for the general formula (1) and X is a halogen atom, and a compound represented by the following general formula (4):

[Chem. 4]

$$W—Z \quad (4)$$

wherein W is the same as defined above, Z is one member selected from the group consisting of -MgCl, -MgBr, -MgI, -ZnCl, -ZnBr, -ZnI, $-Sn(R^3)_3$ (wherein $R^3$ is an alkyl group having 1 to 10 carbon atoms or aryl group that may have a substituent), $-Si(OH)_3$, a boronic acid group, and a boronic acid ester group to a cross-coupling reaction.

It is another preferred embodiment of the present invention to provide a method for producing the thienopyridine derivative, wherein the method comprises subjecting a compound represented by the following general formula (3):

[Chem. 5]

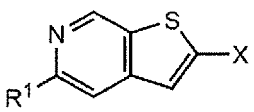

(3)

wherein $R^1$ is the same as defined above for the general formula (1) and X is a halogen atom, and a compound represented by the following formula (5):

[Chem. 6]

$$Z-Y_n-Z \qquad (5)$$

wherein Y is at least one member selected from the group consisting of arylene groups and divalent heteroaromatic ring groups, n is an integer of not less than 1, Z is one member selected from the group consisting of -MgCl, -MgBr, -MgI, -ZnCl, -ZnBr, -ZnI, $-Sn(R^3)_3$ (wherein $R^3$ is an alkyl group having 1 to 10 carbon atoms or aryl group that may have a substituent), $-Si(OH)_3$, a boronic acid group, and a boronic acid ester group to a cross-coupling reaction.

It is another preferred embodiment of the present invention to provide a method for producing the thienopyridine derivative, wherein a compound represented by the following general formula (3):

[Chem. 7]

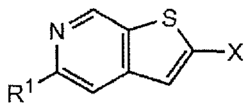

(3)

wherein $R^1$ is the same as defined above for the general formula (1) and X is a halogen atom is lithiated with an organolithium compound and subsequently copper (II) halide is caused to react therewith.

It is another preferred embodiment of the present invention to provide a method for producing a thienopyridine derivative, wherein a compound represented by the following general formula (6):

[Chem. 8]

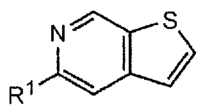

(6)

wherein $R^1$ is the same as defined above for the general formula (1) is caused to react with a halogenating agent to obtain a compound represented by the following general formula (3):

[Chem. 9]

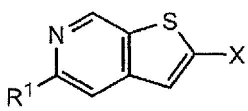

(3)

wherein $R^1$ is the same as defined above for the general formula (1), and X is a halogen atom.

The aforementioned problem can be solved also by providing a compound represented by the following general formula (3):

[Chem. 10]

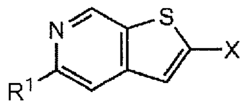

(3)

wherein $R^1$ is a hydrogen atom, an alkyl group that may have a substituent, an alkenyl group that may have a substituent, an alkynyl group that may have a substituent, an alkoxy group that may have a substituent, an aryl group that may have a substituent, a heteroaromatic ring group that may have a substituent, an alkylthio group that may have a substituent, an arylthio group that may have a substituent, an ester group that may have a substituent, or a substituent represented by $-SO_2R^2$ (wherein $R^2$ is a hydrocarbon group having 1 to 20 carbon atoms that may have a substituent), and X is a halogen atom.

In the above, an organic semiconductor device containing the thienopyridine derivative of the present invention is one preferred embodiment of the present invention, and it is preferred that the organic semiconductor device is an organic thin film transistor element.

## EFFECT OF THE INVENTION

According to the present invention, a novel thienopyridine derivative and a method for the production thereof can be provided. The thus-obtained thienopyridine derivative is useful as an organic semiconductor device, and particularly, it is preferably used as an organic thin film transistor element.

## BRIEF DESCRIPTION OF THE DRAWINGS

[Fig. 1] A schematic diagram illustrating the constitution of an organic thin film transistor produced in Example 7.

## MODE FOR CARRYING OUT THE INVENTION

According to the present invention, a thienopyridine derivative represented by a general formula (1) and a method for the production thereof can be provided. The thienopyridine derivative represented by the general formula (1) is a novel compound. Details are described below.

[Chem. 11]

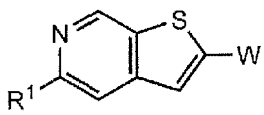

(1)

In the formula, $R^1$ is a hydrogen atom, an alkyl group that may have a substituent, an alkenyl group that may have a substituent, an alkynyl group that may have a substituent, an alkoxy group that may have a substituent, an aryl group that may have a substituent, a heteroaromatic ring group that may have a substituent, an alkylthio group that may have a substituent, an arylthio group that may have a substituent, an ester group that may have a substituent, or a substituent represented by $-SO_2R^2$ (wherein $R^2$ is a hydrocarbon group having 1 to 20 carbon atoms that may have a substituent), and W is at least one member selected from the group consisting of aryl groups that may have a substituent and heteroaromatic ring groups that may have a substituent.

In the general formula (1) , $R^1$ is a hydrogen atom, an alkyl group that may have a substituent, an alkenyl group that may have a substituent, an alkynyl group that may have a substituent, an alkoxy group that may have a substituent, an aryl group that may have a substituent, a heteroaromatic ring group that may have a substituent, an alkylthio group that may have a substituent, an arylthio group that may have a substituent, an ester group that may have a substituent, or a substituent represented by $-SO_2R^2$ ($R^2$ is a hydrocarbon group having 1 to 20 carbon atoms that may have a substituent).

The alkyl group to be used for $R^1$ in the general formula (1) of the present invention may be a linear or branched alkyl group or alternatively may be a cyclic cycloalkyl group; examples thereof include linear or branched alkyl groups such as a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, an isohexyl group, a 2-ethylhexyl group, a heptyl group, an octyl group, a nonyl group, a decyl group, and a dodecyl group; and cycloalkyl groups such as a cyclopropyl group, a cyclobutyl group, a cyclopentyl group, a cyclohexyl group, a cycloheptanyl group, a cyclooctanyl group, a cyclononanyl group, a cyclodecanyl group, a cycloundecanyl group, and a cyclododecanyl group.

The alkyl group in the above $R^1$ may have a substituent and examples of such a substituent include aryl groups, such as a phenyl group, a naphthyl group, an anthryl group, and a phenanthryl group; heteroaromatic ring groups, such as a pyridyl group, a thienyl group, a furyl group, a pyrrolyl group, an imidazolyl group, a pyrazinyl group, an oxazolyl group, an thiazolyl group, a pyrazolyl group, a benzothiazolyl group, and a benzoimidazolyl group; alkoxy groups, such as a methoxy group, an ethoxy group, a propoxy group, an isopropoxy group, a butoxy group, an isobutoxy group, a sec-butoxy group, a tert-butoxy group, a pentyloxy group, an isopentyloxy group, a neopentyloxy group, a hexyloxy group, a cyclohexyloxy group, a heptyloxy group, an octyloxy group, a nonyloxy group, a decyloxy group, and a dodecyloxy group; alkylthio groups, such as a methylthio group, an ethylthio group, a propylthio group, and a butylthio group; arylthio groups, such as a phenylthio group and a naphthylthio group; tri-substituted silyloxy groups, such as a tert-butyldimethylsilyloxy group and a tert-butylphenylsilyloxy group; acyloxy groups, such as an acetoxy group, a propanoyloxy group, a butanoyloxy group, a pivaloyloxy group, and a benzoyloxy group; alkoxycarbonyl groups, such as a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, a hexyloxycarbonyl group, a heptyloxycarbonyl group, and an octyloxycarbonyl group; alkylsulfinyl groups, such as a methylsulfinyl group and an ethylsulfinyl group; arylsulfinyl groups, such as a phenylsulfinyl group; sulfonic acid ester groups, such as a methylsulfonyloxy group, an ethylsulfonyloxy group, a phenylsulfonyloxy group, a methoxysulfonyl group, an ethoxysulfonyl group, and a phenyloxysulfonyl group; an amino group; a hydroxy group; a cyano group; a nitro group; and halogen groups, such as a fluorine atom, a chlorine atom, a bromine atom, and an iodine atom.

The amino group out of the substituents which the above $R^1$ may have may be a secondary amino group or a tertiary amino group as well as a primary amino group ($-NH_2$). The secondary amino group is a monosubstituted amino group represented by $-NHR^4$ ($R^4$ is an arbitrary monovalent substituent), and examples of $R^4$ include alkyl groups, aryl groups, an acetyl group, a benzoyl group, a benzenesulfonyl group, and a tert-butoxycarbonyl group. Specific examples of the secondary amino group include secondary amino groups in which $R^4$ is an alkyl group, such as a methylamino group, an ethylamino group, a propylamino group, and an isopropylamino group, and secondary amino groups in which $R^4$ is an aryl group, such as a phenylamino group and a naphthylamino group. A hydrogen atom of the alkyl group or the aryl group in $R^4$ may be further substituted with an acetyl group, a benzoyl group, a benzenesulfonyl group, a tert-butoxycarbonyl group, or the like. The tertiary amino group is a di-substituted amino group represented by $-NR^4R^5$ ($R^4$ and $R^5$ are at least one member selected from the group consisting of alkyl groups and aryl groups); groups similar to those of $R^4$ may be used as $R^5$, and $R^4$ and $R^5$ may be the same or different from each other. Specific examples of the tertiary amino group include tertiary amino groups in which $R^4$ and $R^5$ are selected from alkyl groups and aryl groups, such as a dimethylamino group, a diethylamino group, a dibutylamino group, an ethylmethylamino group, a diphenylamino group, and a methylphenylamino group.

The alkenyl group to be used in $R^1$ of the general formula (1) of the present invention may be either a linear chain or a branched chain. Examples of the alkenyl group include a vinyl group, an allyl group, a methylvinyl group, a propenyl group, a butenyl group, a pentenyl group, a hexenyl group, a cyclopropenyl group, a cyclobutenyl group, a cyclopentenyl group and a cyclohexenyl group. Such alkenyl groups may have a substituent, and as such a substituent, substituents the same as those provided as examples in the explanation of the alkyl group can be used.

The alkynyl group to be used in $R^1$ of the general formula (1) of the present invention may be either a linear chain or a branched chain. Examples of the alkynyl group include an ethynyl group, a propynyl group, a propargyl group, a butynyl group, a pentynyl group, a hexynyl group, and a phenylethynyl group. Such alkynyl groups may have a substituent, and as such a substituent, substituents the same as those provided as examples in the explanation of the alkyl group can be used.

Examples of the alkoxy group to be used in $R^1$ of the general formula (1) of the present invention include a methoxy group, an ethoxy group, a n-propoxy group, an isopropoxy group, a n-butoxy group, an isobutoxy group, a sec-butoxy group, a tert-butoxy group, a n-pentyloxy group, an isopentyloxy group, a neopentyloxy group, a n-hexyloxy group, an isohexyloxy group, a 2-ethylhexyloxy group, a n-heptyloxy group, a n-octyloxy group, a n-nonyloxy group, and a n-decyloxy group. Such alkoxy groups may have a substituent, and as such a substituent, substituents the same as those, excluding alkoxy groups, provided as examples in the explanation of the alkyl group can be used.

Examples of the aryl group to be used in $R^1$ of the general formula (1) of the present invention include a phenyl group, a naphthyl group, an anthryl group and a phenanthryl group. Such aryl groups may have a substituent, and as

such a substituent, the aforementioned alkyl groups, alkenyl groups, alkynyl groups, and substituents the same as those, excluding aryl groups, provided as examples in the explanation of the alkyl group can be used.

Examples of the heteroaromatic ring group to be used in $R^1$ of the general formula (1) of the present invention include a pyridyl group, a thienyl group, a furyl group, a pyrrolyl group, an imidazolyl group, a pyrazinyl group, an oxazolyl group, a thiazolyl group, a pyrazolyl group, a benzothiazolyl group, and a benzoimidazolyl group. Such heteroaromatic ring groups may have a substituent, and as such a substituent, the aforementioned alkyl groups, alkenyl groups, alkynyl groups, and substituents the same as those, excluding heteroaromatic ring groups, provided as examples in the explanation of the alkyl group can be used.

Examples of the alkylthio group to be used in $R^1$ of the general formula (1) of the present invention include a methylthio group, an ethylthio group, a propylthio group, and a butylthio group. Examples of the arylthio group to be used in the present invention include a phenylthio group and a naphthylthio group. Such alkylthio groups and arylthio groups may have a substituent, and as such a substituent, substituents the same as those, excluding alkylthio groups or arylthio groups, provided as examples in the explanation of the alkyl group can be used.

Examples of the ester group to be used in $R^1$ of the general formula (1) of the present invention include alkoxycarbonyl groups, such as a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, a hexyloxycarbonyl group, a heptyloxycarbonyl group, and an octyloxycarbonyl group. Such alkoxycarbonyl groups may have a substituent, and as such a substituent, substituents the same as those, excluding alkoxycarbonyl groups, provided as examples in the explanation of the alkyl group can be used.

The substituent represented by $-SO_2R^2$ ($R^2$ is a hydrocarbon group having 1 to 20 carbon atoms that may have a substituent) to be used in $R^1$ of the general formula (1) of the present invention is not particularly limited. In the above, $R^2$ is a hydrocarbon group having 1 to 20 carbon atoms which may have a substituent; examples of the hydrocarbon group having 1 to 20 carbon atoms that may have a substituent include alkyl groups that may have a substituent, alkenyl groups that may have a substituent, alkynyl groups that may have a substituent, and aryl groups that may have a substituent. As the alkyl group that may have a substituent, the alkenyl group that may have a substituent, the alkynyl group that may have a substituent, and the aryl group that may have a substituent, substituents the same those provided as examples in the explanation of $R^1$ described above can be used. Particularly, from the viewpoint of availability of raw materials, $R^2$ is preferably an alkyl group that may have a substituent or an aryl group that may have a substituent, and it is more preferably an aryl group that may have a substituent. Specific examples of the substituent represented by $-SO_2R^2$ include alkylsulfonyl groups, such as a methylsulfonyl group and an ethylsulfonyl group; and arylsulfonyl groups, such as a phenylsulfonyl group and a 4-methylphenylsulfonyl group. Such alkylsulfonyl groups and arylsulfonyl groups may have a substituent, and as such a substituent, substituents the same as those provided as examples in the explanation of the alkyl group can be used.

In the thienopyridine derivative represented by the general formula (1), it is preferred from the viewpoint of synthesizing method that $R^1$ is a hydrogen atom, an alkyl group that may have a substituent, an alkoxy group that may have a substituent, an aryl group that may have a substituent, a heteroaromatic ring group that may have a substituent, an ester group that may have a substituent, or a substituent represented by $-SO_2R^2$ ($R^2$ is the same as defined above). The number of carbon atoms of $R^1$ is preferably 1 to 20 including the carbon atoms of the substituent which $R^1$ may have.

In the general formula (1), W is at least one member selected from the group consisting of aryl groups that may have a substituent and heteroaromatic ring groups that may have a substituent. The substituent which the aryl group or the heteroaromatic ring group may have can also be an aryl group or a heteroaromatic ring group. That is, W may be either an single substituent that consists of one member selected from the group consisting of aryl groups that may have a substituent and heteroaromatic ring groups that may have a substituent, or a substituent in which combined two or more groups of at least one member selected from the group consisting of aryl groups that may have a substituent and heteroaromatic ring groups that may have a substituent.

As the aryl group that may have a substituent to be used for W, substituents the same those provided as examples in the explanation of $R^1$ described above can be used. As the heteroaromatic ring group that may have a substituent to be used in W, substituents the same as those provided as examples in the explanation of $R^1$ described above and heteroaromatic ring groups represented by the following general formula (2) can be used. Particularly, from the viewpoints of the availability of raw materials and ease of synthesis, such a substituent as a thienyl group or a bithienyl or terthienyl group in which two or more thienyl groups are combined to each other, or a heteroaromatic ring group represented by the following general formula (2) is preferably used, a substituent in which two or more thienyl groups are combined to each other or a heteroaromatic ring group represented by the following general formula (2) is more preferably used, and a heteroaromatic ring group represented by the following general formula (2) is even more preferably used.

[Chem. 12]

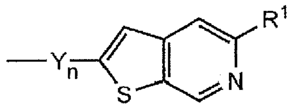

(2)

In the formula, $R^1$ is the same as defined for the general formula (1), Y is at least one member selected from the group consisting of arylene groups and divalent heteroaromatic ring groups, and n is an integer of not less than 0.

In the general formula (2), Y is at least one member selected from the group consisting of arylene groups and divalent heteroaromatic ring groups, and n is an integer of not less than 0. When n is 0, a thienopyridine derivative represented by the general formula (1) in which a substituent represented by the general formula (2) is directly bonded to the 2-position of the thiophene ring without intervention of Y is obtained as understood from Example 1 described below. On the other hand, when n is an integer of not less than 1, a thienopyridine derivative represented by the general formula (1) in which a substituent represented by the general formula (2) is bonded to the 2-position of the thiophene ring with intervention of Y which is at least one member selected from the group consisting of arylene groups and divalent heteroaromatic ring groups is obtained as understood from Examples 2 to 5 described below.

Examples of the arylene group to be used for Y in the general formula (2) include phenylene, 2,3-dialkylphenylene, 2,5-dialkylphenylene, 2,3,5,6-tetraalkylphenylene, 2,3-alkoxyphenylene, 2,5-alkoxyphenylene, 2,3,5,6-tetraalkoxyphenylene, 2-(N,N-dialkylamino)phenylene, 2,5-di(N,N-dialkylamino)phenylene, 2,3-di(N,N-dialkylamino) phenylene, p-phenyleneoxide, p-phenylenesulfide, p-phenyleneamino, p-phenylenevinylene, fluorenylene, naphthylene, anthrylene, tetracenylene, pentacenylene, hexacenylene, heptacenylene, naphthylenevinylene, perinaphthylene, aminopyrenylene, and phenanthrenylene, and at least one member selected from these is preferably used.

Examples of the divalent heteroaromatic ring group to be used for Y include carbazole derivatives, such as N-alkylcarbazoles; pyridine derivatives, such as pyridine, pyrimidine, pyridazine, triazine, pyrazine, quinoline, and purine; furan derivatives, such as furan and 3-alkylfuran; pyrrole derivatives, such as pyrrole, N-alkylpyrroles, ethylene-3,4-dioxypyrrole, and propylene-3,4-dioxypyrrole; thiophene derivatives, such as thiophene, thiophenevinylene, alkylthiophenes, ethylene-3,4-dioxythiophene, propylene-3,4-dioxythiophene, thienothiophene, thienofuran, thienopyrazine, and isothianaphthene; heterocyclic ring derivatives, such as oxadiazole, thiazyl, selenophene, tellurophene, imidazole, oxazole, thiazole, pyrazole, isoxazole, isothiazole, benzotriazole, pyrane, benzothiadiazole, and benzoxadiazole, and at least one member selected from these is preferably used. Particularly, thiophene derivatives are more preferably used.

In the present invention, the thienopyridine derivative represented by the general formula (1) can be obtained by introducing a halogen atom into the 2-position of the thiophene ring of a compound represented by a general formula (6), thereby obtaining a compound represented by a general formula (3), and then causing the compound represented by the general formula (3) and a compound represented by a general formula (4) to undergo a cross-coupling reaction like reactions 1 and 2 shown by the following chemical reaction formula (I).

[Chem. 13]

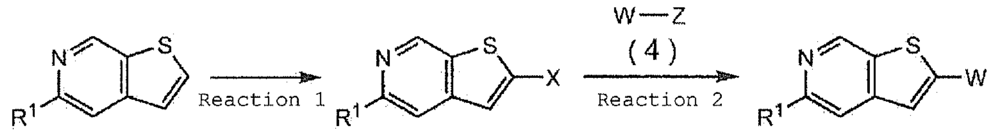


(I)

(6) (3) (1)

In the formula, $R^1$ and W are the same as defined for the general formula (1), X is a halogen atom, and Z is one member selected from the group consisting of -MgCl, -MgBr, -MgI, -ZnCl,- ZnBr, -ZnI, $-Sn(R^3)_3$ (wherein $R^3$ is an alkyl group having 1 to 10 carbon atoms or aryl group that may have a substituent), $-Si(OH)_3$, a boronic acid group, and a boronic acid ester group.

X in the compound represented by the general formula (3) is a halogen atom and examples of the halogen atom include a fluorine atom, a chlorine atom, a bromine atom, and an iodine atom. Particularly, a bromine atom or an iodine atom is preferably used in view of the reactivity of the coupling reaction. Z in the compound represented by the general formula (4) is at least one member selected from the group consisting of -MgCl, -MgBr, -MgI, -ZnCl, -ZnBr, -ZnI, $-Sn(R^3)_3$ (wherein $R^3$ is an alkyl group having 1 to 10 carbon atoms or aryl group that may have a substituent), $-Si(OH)_3$, a boronic acid group, and a boronic acid ester group; from the viewpoint that synthesis efficiency is good, at least one member selected from the group consisting of $-Sn(R^3)_3$, a boronic acid group, and a boronic acid ester group is preferably

used. In the above, $R^3$ is an alkyl group having 1 to 10 carbon atoms that may have a substituent or an aryl group that may have a substituent; as such an alkyl group having 1 to 10 carbon atoms that may have a substituent or an aryl group that may have a substituent, alkyl groups having 1 to 10 carbon atoms and aryl groups out of the alkyl groups and aryl groups provided as examples in the preceding explanation of $R^1$ described above can be used.

A preferred embodiment of the introduction of a halogen atom in the reaction 1 may be a method in which a halogenating agent is caused to react in the presence of a solvent under an atmosphere of an inert gas such as nitrogen and argon. Examples of the solvent to be used in the reaction 1 include hydrocarbons, such as benzene, toluene, xylene, cumene, hexane, heptane, and octane; halogenated hydrocarbons, such as dichloromethane, chloroform, carbon tetrachloride, 1,2-dichloroethane, 1,1,2,2-tetrachloroethane, and chlorobenzene; ethers, such as diethyl ether, diisopropyl ether, dibutyl ether, tetrahydrofuran, dioxane, and dimethoxyethane; esters, such as ethyl acetate and butyl acetate; nitriles, such as acetonitrile and propionitrile; and amides, such as dimethylformamide and N-methylpyrrolidone. A solvent may be used singly or two or more solvents may be used in combination. The amount of such a solvent to be used is preferably from 1 to 100 parts by mass, and more preferably from 1 to 50 parts by mass relative to 1 part by mass of the compound represented by the general formula (6).

Examples of the halogenating agent to be used in the reaction 1 include chlorinating agents, such as N-chlorosuccinimide, N-chlorophthalimide, chlorine, phosphorus pentachloride, thionyl chloride, and 1,2-dichloro-1,1,2,2-tetrafluoroethane; brominating agents, such as N-bromosuccinimide, N-bromophthalimide, N-bromoditrifluoromethylamine, bromine, boron tribromide, copper bromide, silver bromide, tert-butyl bromide, bromine oxide, and 1,2-dibromo-1,1,2,2-tetrafluoroethane; and iodinating agents, such as iodine, iodine trichloride, N-iodophthalimide, and N-iodosuccinimide. Among these, use of a brominating agent or an iodinating agent is preferred and use of a brominating agent is more preferred. The amount of the halogenating agent to be used in the reaction 1 is not particularly limited and it is preferably from 0.5 to 5 mol relative to 1 mol of the compound represented by the general formula (6). When the amount of the halogenating agent to be used exceeds 5 mol, the operation to remove the unreacted halogenating agent may become complicated and it is more preferably not more than 4 mol. On the other hand, when the amount of the halogenating agent to be used is less than 0.5 mol, the operation of separation and purification from the unreacted compound represented by the general formula (6), which is a raw material, may become complicated and it is more preferably not less than 1 mol.

In the reaction 1, the reaction temperature at which the compound represented by the general formula (6) and the halogenating agent are caused to react is not particularly limited, and it is preferably within the range of from -100 to 200°C. When the reaction temperature is lower than -100°C, the rate of reaction may become extremely slow, and it is more preferably not lower than -50°C. On the other hand, when the reaction temperature exceeds 200°C, there is a possibility of promoting the decomposition of a product or a halogenating agent, and it is more preferred to be not higher than 100°C. The reaction time is preferred to be from 1 minute to 20 hours, and more preferred to be from 0.5 to 10 hours. The reaction pressure is preferably from 0 to 3 MPa (gauge pressure).

Subsequently, a thienopyridine derivative represented by the general formula (1) can be obtained by causing a compound represented by the general formula (3) and a compound represented by the general formula (4) to undergo a cross-coupling reaction as shown by the reaction 2. One example of a preferred embodiment of the reaction 2 is a method in which a compound represented by the general formula (3) obtained by the reaction 1 and a compound represented by the general formula (4) are caused to undergo a cross-coupling reaction in the presence of a solvent and a metal complex under an atmosphere of an inert gas such as nitrogen and argon. Examples of the metal complex to be used in the reaction 2 include complexes of metals such as nickel and palladium; particularly, use of a metal complex in which a phosphine coordinates, such as $Pd(PPh_3)_4$ and $NiCl_2(dppp)$, is preferred.

Examples of the cross-coupling reaction in the reaction 2 include the Stille coupling reaction, the Kumada-Tamao coupling reaction, the Hiyama coupling reaction, the Suzuki coupling reaction, the Yamamoto reaction, the Kumada-Corriu reaction, the Riecke reaction, and the McCullogh reaction. Particularly, from the viewpoint of reaction efficiency, the Stille coupling reaction, the Suzuki coupling reaction, the Kumada-Tamao coupling reaction, or the Hiyama coupling reaction is preferably employed, and the Stille coupling reaction and the Suzuki coupling reaction are more preferably employed. The Stille coupling reaction is a reaction using an organotin compound that is a compound represented by the general formula (4) wherein Z is a group represented by $-Sn(R^3)_3$; the Suzuki coupling reaction is a reaction using an organoboron compound that is a compound represented by the general formula (4) wherein Z is a boronic acid group and a boronic acid ester group; the Kumada-Tamao coupling reaction is a reaction using an organomagnesium compound that is a compound represented by the general formula (4) wherein Z is a group represented by -MgBr or the like; and the Hiyama coupling reaction is a reaction using an organosilicon compound that is a compound represented by the general formula (4) wherein Z is a group represented in $-Si(OH)_3$ or the like.

In the reaction 2, a solvent, such as an organic solvent or water, is usually used, and an organic solvent is preferably used. Examples of the organic solvent to be used include alcohols, such as methanol and ethanol; aprotic polar solvents, such as N-methylpyrrolidone, N,N-dimethylformamide, N,N-dimethylacetamide, dimethyl sulfoxide, and acetonitrile; ethers, such as diethyl ether, diisopropyl ether, ethylene glycol dimethyl ether, diethylene glycol dimethyl

ether, 1,4-dioxane, and tetrahydrofuran; aromatic hydrocarbons, such as benzene, toluene, and xylene; aliphatic hydrocarbons, such as hexane and heptane; particularly, an aprotic solvent or an ether is preferably used. Such solvents may be used singly or two or more solvents may be used in combination. The amount of such an organic solvent to be used is preferably from 1 to 200 parts by mass, and more preferably from 5 to 100 parts by mass relative to 1 part by mass of the compound represented by the general formula (3).

Although the reaction temperature in the cross-coupling reaction of the reaction 2 is not particularly limited, it is preferably within the range of from -50 to 200°C. When the reaction temperature is lower than -50°C, the rate of reaction may become extremely slow, and it is more preferably not lower than -20°C. On the other hand, when the reaction temperature exceeds 200°C, there is a possibility of promoting the decomposition of a product or a metal complex serving as a catalyst, and it is more preferred to be not higher than 100°C. The reaction time is preferred to be from 1 minute to 80 hours, and more preferred to be from 0.5 to 60 hours. The reaction pressure is preferably from 0 to 3 MPa (gauge pressure).

In the present invention, the compound represented by the following general formula (1a), which is one preferred embodiment of the compound represented by the general formula (1), can be obtained by causing a compound represented by the general formula (3) and a compound represented by the general formula (5) to undergo a cross-coupling reaction as in a reaction 3 represented by the following chemical reaction formula (II).

[Chem. 14]

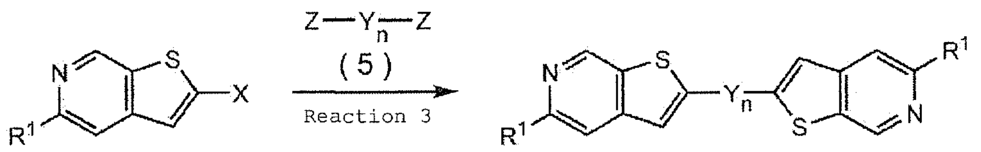


(3) (1a) (II)

In the formula, $R^1$ is the same as defined for the general formula (1), X is a halogen atom, and Y is at least one member selected from the group consisting of arylene groups and divalent heteroaromatic ring groups, n is an integer of not less than 1, Z is one member selected from the group consisting of -MgCl, -MgBr, -MgI, -ZnCl, -ZnBr, -ZnI, $-Sn(R^3)_3$ (wherein $R^3$ is an alkyl group having 1 to 10 carbon atoms or aryl group that may have a substituent), $-Si(OH)_3$, a boronic acid group, and a boronic acid ester group.

The reaction 3 is a reaction to obtain a compound represented by the general formula (1a) by causing a compound represented by the general formula (3) and a compound represented by the general formula (5) to undergo a cross-coupling reaction. The thus-obtained compound represented by the general formula (1a) is a compound of the case that W in the compound represented by the general formula (1) is a heteroaromatic ring group represented by the general formula (2). Here, the reactions provided as examples in the explanation of the reaction 2 can be adopted as the cross-coupling reaction. Y in the thus-obtained thienopyridine derivative represented by the general formula (1a) is at least one member selected from the group consisting of arylene groups and divalent heteroaromatic ring groups, and groups the same as those provided as examples in the explanation of the general formula (2) are preferably employed.

In the present invention, the compound represented by the following general formula (1b), which is one preferred embodiment of the compound represented by the general formula (1), can be obtained by lithiating a compound represented by the general formula (3) with an organolithium compound and then causing copper (II) halide to react as in a reaction 4 represented by the following chemical reaction formula (III).

[Chem. 15]

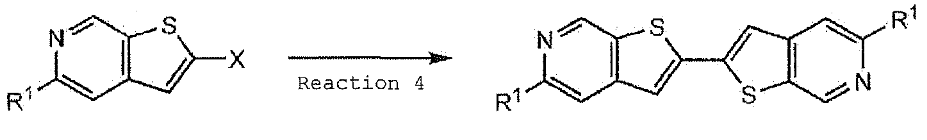


(3) (1b) (III)

In the formula, $R^1$ is the same as defined for the general formula (1) and X is a halogen atom.

The organolithium compound to be used in the reaction 4 is not particularly limited and, for example, alkyllithium

compounds, such as methyllithium, n-butyllithium, sec-butyllithium, and tert-butyllithium; aryllithium compounds, such as phenyllithium; alkenyllithium compounds, such as vinyllithium; and lithium amide compounds, such as lithium diisopropylamide and lithium bistrimethylsilylamide are used. Among these, it is preferred to use an alkyllithium compound. The amount of the organolithium compound to be used is not particularly limited and it is preferably from 0.5 to 5 mol relative to 1 mol of the compound represented by the general formula (3). When the used amount of the organolithium compound exceeds 5 mol, there is a possibility of promoting a side reaction or the decomposition of a product, and it is more preferred to be 4 mol or less. It is more preferred that the used amount of the organolithium compound is not less than 1 mol.

The copper (II) halide to be used in the reaction 4, is not particularly limited and examples thereof include copper (II) chloride, copper (II) bromide, and copper (II) iodide. The amount of the copper (II) halide to be used is not particularly limited and it is preferably from 0.5 to 5 mol relative to 1 mol of the compound represented by the general formula (3). When the amount of the copper (II) halide to be used exceeds 5 mol, the operation to remove excess copper (II) halide may become complicated and it is more preferably not more than 4 mol. The amount of the copper (II) halide to be used is more preferably not less than 1 mol.

It is preferred that the reaction 4 is carried out in the presence of a solvent. Examples of such a solvent include saturated aliphatic hydrocarbons, such as pentane, hexane, heptane, octane, nonane, decane, and cyclohexane; aromatic hydrocarbons, such as benzene, toluene, ethylbenzene, propylbenzene, xylene, and ethyltoluene; ethers, such as dimethyl ether, ethyl methyl ether, diethyl ether, dipropyl ether, butyl methyl ether, tert-butyl methyl ether, dibutyl ether, tetrahydrofuran, and 1,4-dioxane; aprotic polar solvents, such as dimethylacetamide, dimethylformamide, N-methyl-2-pyrrolidone, and dimethyl sulfoxide. Among these, use of an ether is preferred; specifically, use of tetrahydrofuran, 1,4-dioxane, or diethyl ether is preferred. A solvent may be used singly or two or more solvents may be used in combination. The amount of such a solvent to be used is preferably from 1 to 100 parts by mass, and more preferably from 5 to 50 parts by mass relative to 1 part by mass of the compound represented by the general formula (3).

Although the reaction temperature in the reaction 4 is not particularly limited, it is preferably within the range of from -100 to 25°C. When the reaction temperature is lower than -100°C, the rate of reaction may become extremely slow, and it is more preferably not lower than -90°C. On the other hand, when the reaction temperature exceeds 25°C, the decomposition of a product may be promoted and the reaction temperature is more preferably not higher than 20°C. The reaction time is preferred to be from 1 minute to 10 hours, and more preferred to be from 5 minutes to 5 hours.

The thienopyridine derivative of the present invention represented by the general formula (1) described above can be preferably used as a new organic semiconductor material. Particularly, it is preferably used as an organic semiconductor device, such as an organic thin film transistor element, a photoelectric conversion device, an organic electroluminescent element, and a liquid crystal display element, and it is more preferably used as an organic thin film transistor element. An embodiment of an organic thin film transistor element using the thienopyridine derivative of the present invention is described below.

In the organic thin film transistor element of the present invention, using an organic semiconductor layer containing the thienopyridine derivative represented by the general formula (1), a substrate, a gate electrode, a gate insulating layer, a source-drain electrode, and so on, and, for example, a configuration of substrate/gate electrode/gate insulating layer/organic semiconductor layer/source-drain electrode, a configuration of substrate/gate electrode/gate insulating layer/source-drain electrode/organic semiconductor layer, and so on are preferably employed, but the present invention is not limited thereto.

The organic semiconductor layer that constitutes the organic thin film transistor element of the present invention is preferably formed by forming a thin film of the thienopyridine derivative of the present invention, and examples of the thin film method include a vacuum deposition method, a spin coating method, a casting method, an LB method, a screen printing method, a printing method, a dipping method, and an inkjet method.

The substrate that constitutes the organic thin film transistor element of the present invention, is not particularly limited and examples thereof include glass, polyethylene naphthalate (PEN), polyethylene terephthalate (PET), polycarbonate, poly(vinyl alcohol), polyacrylate, polyimide, poly norbornene, and polyethersulfone (PES).

The gate electrode and the source-drain electrode that constitute the organic thin film transistor element of the present invention are not particularly limited as far as they are made of metal that is ordinarily used, and gold, silver, aluminum, nickel, indium tin oxide (ITO), etc. are suitably used therefor.

The gate insulating layer that constitutes the organic thin film transistor element of the present invention is not particularly limited, and insulators with high dielectric constants that are ordinarily used can be used therefor. Specifically, ferroelectricity insulators, such as $Ba_{0.33}Sr_{0.66}TiO_3$ (BST), $Al_2O_3$, $Ta_2O_5$, $La_2O_5$, $Y_2O_3$, and $TiO_2$; inorganic insulators, such as $PbZr_{0.33}Ti_{0.66}O_3$ (PZT), $Bi_4Ti_3O_{12}$, $BaMgF_4$, $SrBi_2(TaNb)_2O_9$, $Ba(ZrTi)O_3$ (BZT), $BaTiO_3$, $SrTiO_3$, $Bi_4Ti_3O_{12}$, $SiO_2$, $SiN_x$, and AlON; organic insulators, such as polyimide, BCB (benzocyclobutene), parylene, polyacrylate, poly (vinyl alcohol), and polyvinylphenol; and so on can be used.

EXAMPLES

The present invention is explained below more concretely by way of Examples.

Example 1

(Synthesis Example 1) [Synthesis of

5-phenylsulfonylthieno[2,3-c]pyridine represented by formula (6a)]

In a three-necked, 300-ml flask equipped with a thermometer, a magnetic stirrer and a dropping funnel, phenyl sulfonylcyanide (3.77 g, 22.6 mmol) and isobutyl chloroformate (3.08 g, 22.6 mmol) were mixed in xylene (100 ml) and stirred during heating under reflux. To the resulting mixture, a solution of 3-methylthiophene-2-carbaldehyde-N-phenylimine (3.03 g, 15 mmol) in xylene (50 ml) was dropped over 30 minutes. After the completion of the dropping, the reaction mixture was heated under reflux for one hour and then allowed to cool to room temperature, and then the solvent was removed by distillation to obtain a crude product. This was purified by silica gel column chromatography, obtaining 2.79 g of 5-phenylsulfonylthieno[2,3-c]pyridine (10.1 mmol, 67% yield). The chemical reaction formula is shown below.
[Chem. 16]

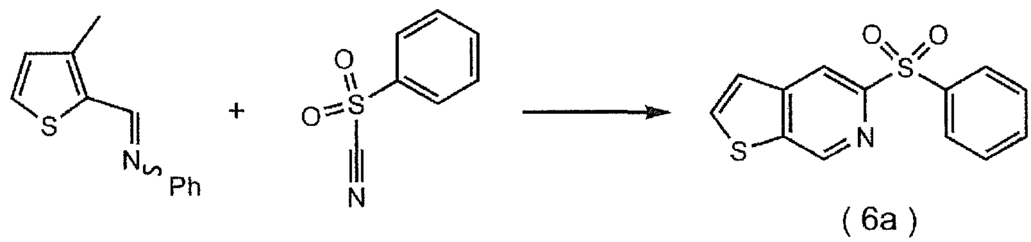

(6a)

The NMR data of 5-phenylsulfonylthieno[2,3-c]pyridine represented by the formula (6a) were as follows.
$^1$H-NMR Spectrum (270MHz, $CDCl_3$, TMS, ppm) δ: 9.16 (d, 1H, J=1.08Hz), 8.66 (d, 1H, J=1.08Hz), 8.13-8.08 (m, 2H), 7.90 (d, 1H, J=4.86Hz), 7.62-7.48 (m, 4H).

(Synthesis Example 2) [Synthesis of

2-bromo-5-phenylsulfonylthieno[2,3-c]pyridine represented by formula (3a)]

To a three-necked, 50-ml flask equipped with a thermometer and a magnetic stirrer, 5-phenylsulfonylthieno [2,3-c]pyridine (2.37 g, 8.6 mmol), N-bromosuccinimide (4.60 g, 25.8 mmol), and 30 ml of acetonitrile were added and then heated at 70°C for 6 hours under stirring. The reaction solution was allowed to cool to room temperature, and then to the residue resulting from the removal of the solvent by distillation were added 100 ml of ethyl acetate and 100 ml of water. The organic layer and the aqueous layer were separated with a separatory funnel, and the aqueous layer was extracted with 50 ml of ethyl acetate twice, which was then mixed with the organic layer separated previously and was washed with 100 ml of water twice, and then the organic layer was dried using anhydrous magnesium sulfate. The crude product resulting from the removal of the solvent by distillation was purified by recrystallization using toluene and ethyl acetate, obtaining 2.49 g of
2-bromo-5-phenylsulfonylthieno[2,3-c]pyridine (7.0 mmol, 81% yield). The chemical reaction formula is shown below.
[Chem. 17]

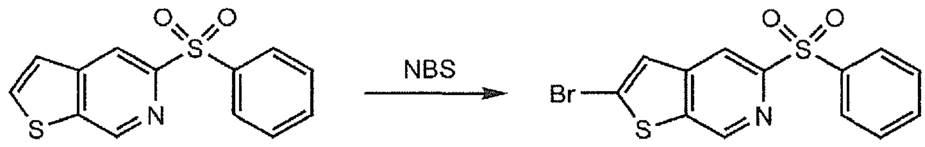


( 6a ) ( 3a )

The NMR data of 2-bromo-5-phenylsulfonylthieno [2, 3-c]pyridine represented by the formula (3a) were as follows. $^1$H-NMR Spectrum (270MHz, $CDCl_3$, TMS, ppm) δ: 9.13 (d, 1H, J=0.81Hz), 8.65 (d, 1H, J=0.81Hz), 8.14-8.10 (m, 2H), 7.86 (s, 1H), 7.63-7.50 (m, 3H).

(Synthesis Example 3-1) [Synthesis of

2-bromo-5-methoxythieno[2,3-c]pyridine represented by formula (3b)]

To a three-necked, 50-ml flask equipped with a thermometer and a magnetic stirrer, 2-bromo-5-phenylsulfonylthieno[2,3-c]pyridine (1.00 g, 2.8 mmol), a 28% methanol solution of sodium methoxide (1. 62 g, 8.4 mmol), and 20 ml of tetrahydrofuran were added and then heated at 60°C for 6 hours under stirring. The reaction solution was allowed to cool to room temperature and 1 ml of acetic acid was added thereto, and then to the residue resulting from the removal of the solvent by distillation were added 50 ml of ethyl acetate and 50 ml of water. The organic phase and the aqueous phase were separated with a separatory funnel, and the organic phase was dried using anhydrous magnesium sulfate. The crude product resulting from the removal of the solvent by distillation was purified by silica gel column chromatography, obtaining 0.635 g of 2-bromo-5-methoxythieno[2,3-c]pyridine (2.6 mmol, 93% yield). The chemical reaction formula is shown below.
[Chem. 18]

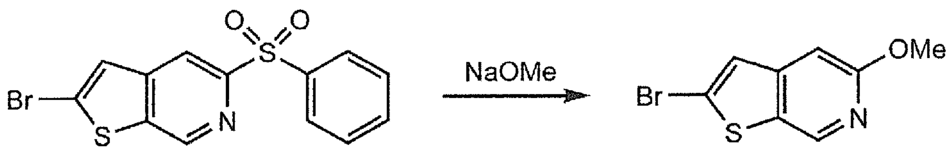


( 3a ) ( 3b )

The NMR data of 2-bromo-5-methoxythieno[2,3-c]pyridine represented by the formula (3b) were as follows. $^1$H-NMR Spectrum (270MHz, $CDCl_3$, TMS, ppm) δ: 8.68 (s, 1H), 7.63 (s, 1H), 7.10 (s, 1H), 4.02 (s, 3H).

(Synthesis Example 3-2) [Synthesis of 2-bromo-5-n-butoxythieno[2,3-c]pyridine represented by formula (3c)]

To a three-necked, 50-ml flask equipped with a thermometer and a magnetic stirrer, 2-bromo-5-phenylsulfonylthieno[2,3-c]pyridine (1.00 g, 2.8 mmol), sodium n-butoxide (806 mg, 8.4 mmol), and 20 ml of tetrahydrofuran were added and then heated at 60°C for 6 hours under stirring. The reaction solution was allowed to cool to room temperature and 1 ml of acetic acid was added thereto, and then to the residue resulting from the removal of the solvent by distillation were added 50 ml of ethyl acetate and 50 ml of water. The organic phase and the aqueous phase were separated with a separatory funnel, and the organic phase was dried using anhydrous magnesium sulfate. The crude product resulting from the removal of the solvent by distillation was purified by silica gel column chromatography, obtaining 0.729 g of 2-bromo-5-n-butoxythieno[2,3-c]pyridine (2.55 mmol, 91% yield). The chemical reaction formula is shown below.
[Chem. 19]

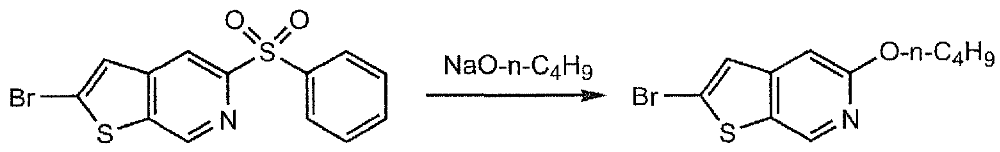


( 3a ) ( 3c )

The NMR data of 2-bromo-5-n-butoxythieno[2,3-c]pyridine represented by the formula (3c) were as follows. $^{1}$H-NMR Spectrum (270MHz, $CDCl_3$, TMS, ppm) δ: 0.99 (t, 3H, J=7.2Hz), 1.51-1.56 (m, 2H), 1.78-1.84 (m, 2H), 4.34 (t, 2H, J=6.5Hz), 7.09 (s, 1H), 7.62 (s, 1H), 8.67 (s, 1H).

(Synthesis Example 3-3) [Synthesis of

2-bromo-5-n-hexyloxythieno[2,3-c]pyridine represented by formula (3d)]

A reaction and post treatment were carried out in the same manner as Synthesis Example 3-2 except that sodium n-butoxide (806 mg, 8.4 mmol) was changed to sodium n-hexyloxide (1.042 g, 8.4 mmol), so that 0.695 g of 2-bromo-5-n-hexyloxythieno[2,3-c]pyridine (2.21 mmol, 79% yield) was obtained. The chemical reaction formula is shown below.

[Chem. 20]

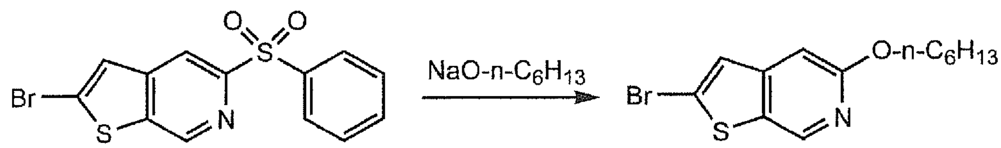


( 3a ) ( 3d )

The NMR data of 2-bromo-5-n-hexyloxythieno[2,3-c]pyridine represented by the formula (3d) were as follows. $^{1}$H-NMR Spectrum (270MHz, $CDCl_3$, TMS, ppm) δ: 0.91 (t, 3H, J=6.8Hz), 1.33-1.37 (m, 4H), 1.45-1.53 (m, 2H), 1.78-1.86 (m, 2H), 4.32 (t, 2H, J=6.5Hz), 7.09 (s, 1H), 7.62 (s, 1H), 8.67 (s, 1H).

(Synthesis Example 4) [Synthesis of

2,2'-bis(5-methoxythieno[2,3-c]pyridine) represented by formula (1b-1)]

To a three-necked, 50-ml flask equipped with a thermometer and a magnetic stirrer, 2-bromo-5-methoxythieno[2,3-c]pyridine (400 mg, 1.64 mmol) and 10 ml of tetrahydrofuran were added and then cooled to -78°C. While the mixed solution was stirred, 1.28 ml (2.13 mmol) of a 1. 66 M hexane solution of n-butyllithium was dropped so that the internal temperature might be kept at not higher than -70°C, and after the dropping the resulting solution was stirred at -78°C for 30 minutes. Copper (II) chloride (264 mg, 1.97 mmol) was added to the reaction liquid and then stirred at -78°C for one hour. After the completion of the reaction, the temperature was increased to room temperature, then 10 ml of 0.1 N aqueous hydrochloric acid solution was added, and the organic phase was separated with a separatory funnel. The aqueous phase was extracted with chloroform, whose mixture with the previously mentioned organic phase was then washed with dilute hydrochloric acid and water in order. After the organic phase was dried using anhydrous magnesium sulfate, the residue resulting from its concentration under reduced pressure was purified by silica gel column chromatography (developing solvent: hexane/ethyl acetate = 95/5), obtaining a target compound, i.e., 2,2'-bis (5-methoxy thieno [2, 3-c] pyridine), as an orange solid (110 mg, 0.34 mmol, 41% yield). The chemical reaction formula is shown below.

[Chem. 21]

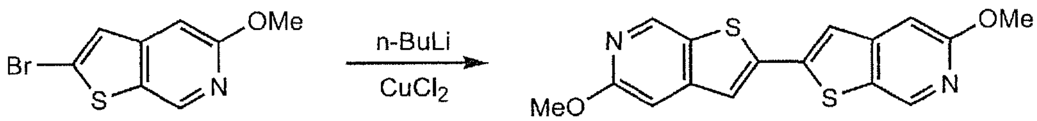


( 3b ) ( 1b-1 )

The NMR data of 2,2'-bis(5-methoxythieno[2,3-c]pyridine) represented by the formula (1b-1) were as follows.
$^{1}$H-NMR Spectrum (270MHz, $CDCl_3$, TMS, ppm) δ: 8.78 (s, 2H), 7.76 (s, 2H), 7.02 (s, 2H), 3.98 (s, 6H).
$^{13}$C-NMR (68.5MHz, $CDCl_3$) δ: 54.1, 102.2, 120.9, 129.5, 141.1, 143.1, 148.7, 162.0.
Mass analysis: m/z 328 ($M^+$)
Melting point: 258°C

Example 2-1

(Synthesis Example 5-1) [Synthesis of 2,5-bis(5-methoxythieno[2,3-c]pyridin-2-yl)thiophene represented by formula (1a-1)]

To a three-necked, 50-ml flask equipped with a thermometer and a magnetic stirrer, 2-bromo-5-methoxythieno[2,3-c]pyridine (200 mg, 0.82 mmol), 2,5-bis(tributylstannyl)thiophene (271 mg, 0.41 mmol), tetrakistriphenylphosphino palladium (48 mg, 0.042 mmol), and 15 ml of N,N-dimethylformamide were added, and after the replacement of the atmosphere in the system with argon, heating was done at 50°C for two days under stirring. After the completion of the reaction, the reaction solution was added to 100 ml of water and then the organic phase was separated with a separatory funnel. The aqueous phase was extracted with methylene chloride, whose mixture with the previously mentioned organic phase was washed with water, and then the organic phase was dried with anhydrous magnesium sulfate. The residue obtained by concentrating the organic phase under reduced pressure was purified by silica gel column chromatography (developing solvent: hexane/ether = 80/20), obtaining a target compound, i.e., 2,5-bis(5-methoxythieno[2,3-c]pyridin-2-yl)thiophene, as an orange solid (58.9 mg, 0.14 mmol, 35% yield based on 2,5-bis(tributylstannyl)thiophene). The chemical reaction formula is shown below.

[Chem. 22]

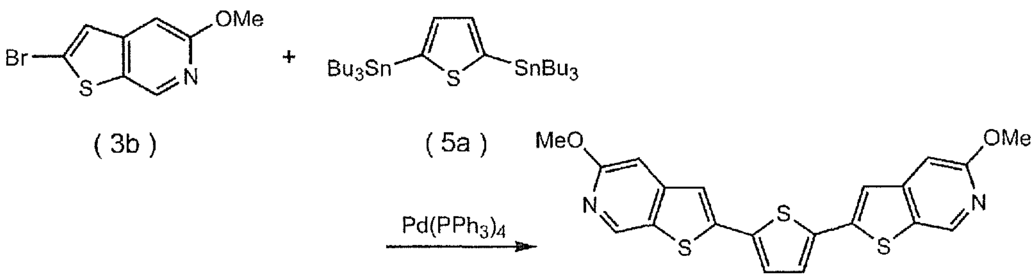


( 3b ) ( 5a ) ( 1a-1 )

The NMR data of 2,5-bis(5-methoxythieno[2,3-c]pyridin-2-yl)thiophene represented by the formula (1a-1) were as follows.
$^{1}$H-NMR Spectrum (270MHz, $CDCl_3$, TMS, ppm) δ: 8.76 (s, 2H), 7.78 (s, 2H), 7.47 (s, 2H), 7.38 (s, 2H), 4.04 (s, 6H).
$^{13}$C-NMR (68MHz, $CDCl_3$, TMS, ppm) δ: 54.24, 101.91, 125.75, 128.92, 130.49, 130.70, 135.79, 141.88, 146.20.

Mass analysis: m/z 410 ($M^+$)
Melting point: 196°C

Example 2-2

(Synthesis Example 5-2) [Synthesis of

2,5-bis(5-n-butoxythieno[2,3-c]pyridin-2-yl)thiophene represented by formula (1a-2)]

A reaction and post treatment were carried out in the same manner as Synthesis Example 5-1 except that 2-bromo-5-methoxythieno[2,3-c]pyridine (200 mg, 0.82 mmol) was changed to 2-bromo-5-n-butoxythieno[2,3-c]pyridine (235 mg, 0.82 mmol), so that 2,5-bis(5-n-butoxythieno[2,3-c]pyridin-2-yl)thiophene was obtained as an orange solid (111.6 mg, 0. 23 mmol, 55% yield based on 2,5-bis(tributylstannyl)thiophene). The chemical reaction formula is shown below.

[Chem. 23]

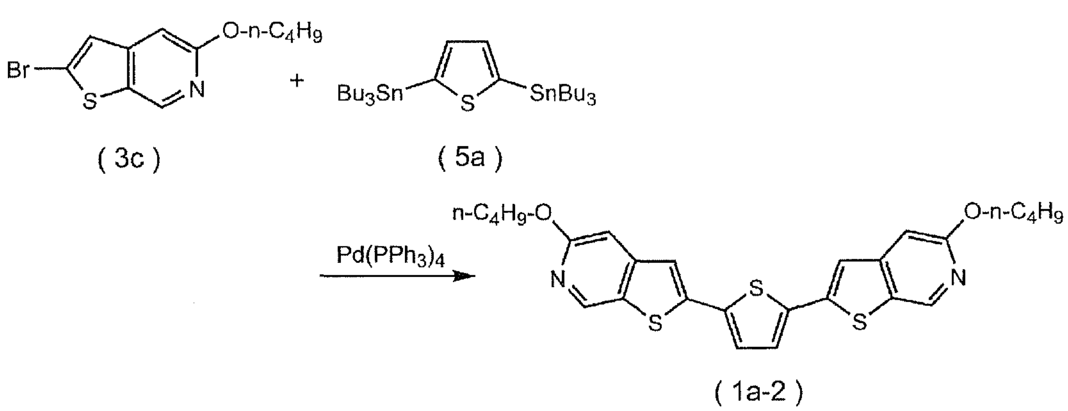


The NMR data of 2,5-bis(5-n-butoxythieno[2,3-c]pyridin-2-yl)thiophene represented by the formula (1a-2) were as follows.
$^1$H-NMR Spectrum (270MHz, $CDCl_3$, TMS, ppm) $\delta$: 0.99 (t, 6H, J=7.2Hz), 1.47-1.55 (m, 4H), 1.77-1.85 (m, 4H), 4.35 (t, 4H, J=6.5Hz), 7.38 (s, 2H), 7.44 (s, 2H), 7.76 (s, 2H), 8.73 (s, 2H).

Example 2-3

(Synthesis Example 5-3) [Synthesis of

2,5-bis(5-n-hexyloxythieno[2,3-c]pyridin-2-yl)thiophene represented by formula (1a-3)]

A reaction and post treatment were carried out in the same manner as Synthesis Example 5-1 except that 2-bromo-5-n-methoxythieno[2,3-c]pyridine (200 mg, 0.82 mmol) was changed to 2-bromo-5-n-hexyloxythieno[2,3-c]pyridine (258 mg, 0.82 mmol), so that 2,5-bis(5-n-hexyloxythieno[2,3-c]pyridin-2-yl)thiophene was obtained as an orange solid (92.6 mg, 0.17 mmol, 41% yield based on 2,5-bis(tributylstannyl)thiophene). The chemical reaction formula is shown below.

[Chem. 24]

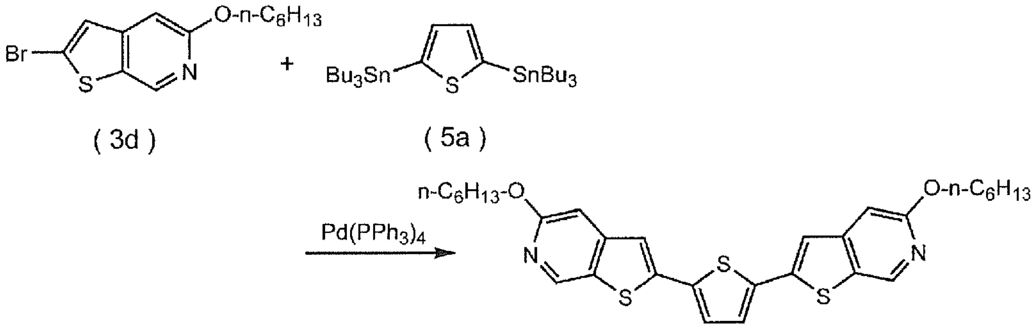


( 3d ) ( 5a )

( 1a-3 )

The NMR data of 2,5-bis(5-n-hexyloxythieno[2,3-c]pyridin-2-yl)thiophene represented by the formula (1a-3) were as follows.
$^1$H-NMR Spectrum (270MHz, $CDCl_3$, TMS, ppm) δ: 0.88-0.91 (t, 6H, J=7.2Hz), 1.33-1.37 (m, 4H, 4H), 1.45-1.52 (m, 4H), 1.78-1.85 (m, 4H), 4.33-4.36 (t, 4H, J=6.5Hz), 7.37 (s, 2H), 7.45 (s, 2H), 7.74 (s, 2H), 8.73 (s, 2H).

Example 2-4

(Synthesis Example 5-4) [Synthesis of

1,4-bis(5-methoxythieno[2,3-c]pyridin-2-yl)benzene represented by formula (1a-4)]

A reaction and post treatment were carried out in the same manner as Synthesis Example 5-1 except that 2,5-bis(tributylstannyl)thiophene (271 mg, 0.41 mmol) was changed to 1, 4-bis (tributylstannyl) benzene (269 mg, 0. 41 mmol), so that 1,4-bis(5-methoxythieno[2,3-c]pyridin-2-yl)benzene was obtained as an orange solid (124.4 mg, 0.308 mmol, 75% yield based on 1,4-bis(tributylstannyl)benzene). The chemical reaction formula is shown below.
[Chem. 25]

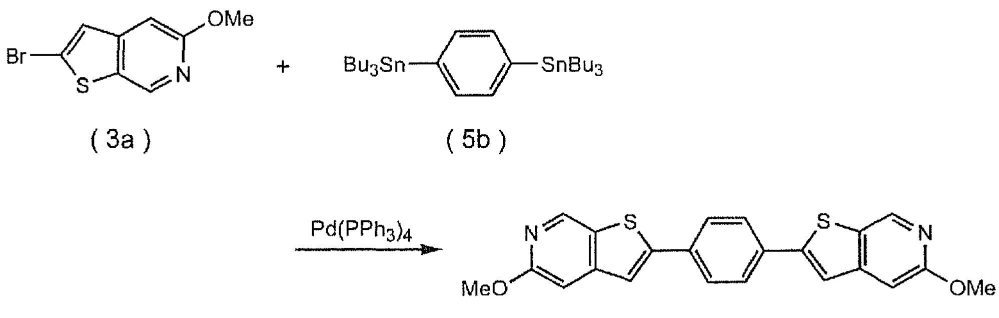


( 3a ) ( 5b )

( 1a-4 )

The NMR data of 1,4-bis(5-methoxythieno[2,3-c]pyridin-2-yl)benzene represented by the formula (1a-4) were as follows.
$^1$H-NMR Spectrum (270MHz, $CDCl_3$, TMS, ppm) δ: 4.02 (s, 6H), 7.29 (s, 2H), 7.68 (s, 6H), 8.77 (s, 2H).

Example 3-1

(Synthesis Example 6-1) [Synthesis of 5,5'-bis(5-methoxythieno[2,3-c]pyridin-2-yl)-2,2'-bithiophe ne represented by formula (1a-5)]

A reaction and purification was carried out in the same manner as Synthesis Example 5-1 except that 2,5-bis (tributylstannyl)thiophene (271 mg, 0.41 mmol) was changed to 5,5'-bis(tributylstannyl)-2,2'-bithiophene (305 mg, 0.41 mmol), so that a target compound, i.e., 5,5'-bis(5-methoxythieno[2,3-c]pyridin-2-yl)-2,2'-bithiophe ne, was obtained as an orange solid (131 mg, 0.27 mmol, 65% yield based on 5,5'-bis(tributylstannyl)-2,2'-bithiophene). The chemical reaction formula is shown below.
[Chem. 26]

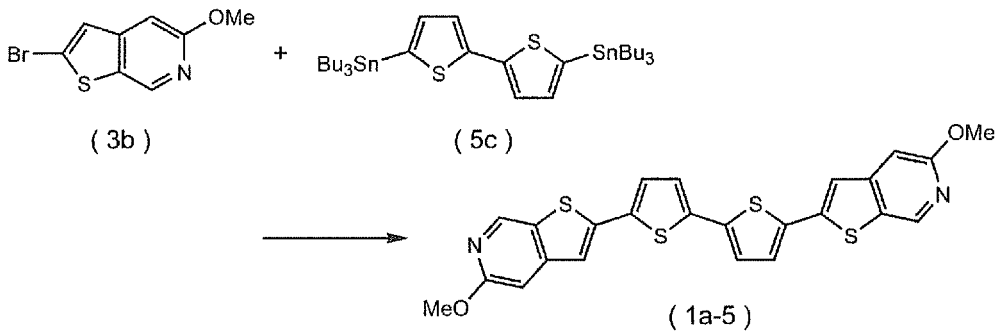

( 3b ) ( 5c ) ( 1a-5 )

The NMR data of
5,5'-bis(5-methoxythieno[2,3-c]pyridin-2-yl)-2,2'-bithiophe ne represented by the formula (1a-5) were as follows.
$^1$H-NMR Spectrum (400MHz, $CDCl_3$) $\delta$: 4. 04 (s, 6H), 7.26 (d, J=3.9Hz, 2H), 7.28 (d, J=3.9Hz, 2H), 7.47 (s, 2H), 7.74 (s, 2H), 8.74 (s, 2H).
$^{13}$C-NMR (67.5MHz, $CDCl_3$) $\delta$: 54.2, 101.9, 124.3, 125.9, 128.9, 130.2, 130.7, 135.2, 136.5, 141.8, 146.1, 162.2.
Mass analysis: m/z 429 ($M^+$)
Melting point: 246°C

Example 3-2

(Synthesis Example 6-2) [Synthesis of

5,5'-bis(5-n-butoxythieno[2,3-c]pyridin-2-yl)-2,2'-bithioph ene represented by formula (1a-6)]

A reaction and post treatment were carried out in the same manner as Synthesis Example 6-1 except that 2-bromo-5-methoxythieno[2,3-c]pyridine (200 mg, 0.82 mmol) was changed to 2-bromo-5-n-butoxythieno[2,3-c]pyridine (235 mg, 0.82 mmol), so that
5,5'-bis(5-n-butoxythieno[2,3-c]pyridin-2-yl)-2,2'-bithioph ene was obtained as an orange solid (137 mg, 0.238 mmol, 58% yield based on 5,5'-bis(tributylstannyl)-2,2'-bithiophene). The chemical reaction formula is shown below.
[Chem. 27]

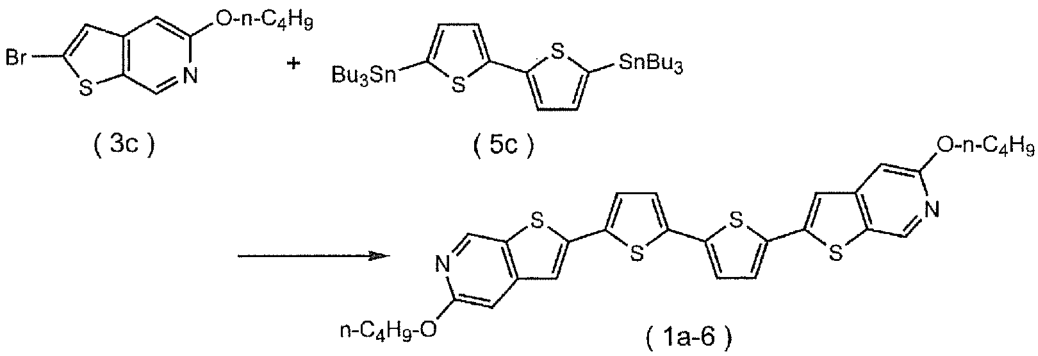

( 3c ) ( 5c ) ( 1a-6 )

The NMR data of 5,5'-bis(5-n-butoxythieno[2,3-c]pyridin-2-yl)-2,2'-bithioph ene represented by the formula (1a-6) were as follows. $^1$H-NMR Spectrum (400 MHz, $CDCl_3$) : 50.99 (t, J=7.2Hz, 6H), 1.50-1.56 (m, 4H), 1.78-1.84 (m, 4H), 4.6 (t, J=6.5 Hz, 4H), 7.26 (d, J=3.9Hz, 2H), 7.28 (d, J=3.9Hz, 2H), 7.28-7.29 (d, J=3.9Hz, 2H), 7.45 (s, 2H), 7.73 (s, 2H), 8.72 (s, 2H).

Example 3-3

(Synthesis Example 6-3) [Synthesis of

5,5'-bis(5-n-hexyloxythieno[2,3-c]pyridin-2-yl)-2,2'-bithio phene represented by formula (1a-7)]

A reaction and post treatment were carried out in the same manner as Synthesis Example 6-1 except that 2-bromo-5-methoxythieno[2,3-c]pyridine (200 mg, 0.82 mmol) was changed to 2-bromo-5-n-hexyloxythieno[2,3-c]pyridine (258 mg, 0.82 mmol), so that
5,5'-bis(5-n-hexyloxythieno[2,3-c]pyridin-2-yl)-2,2'-bithio phene was obtained as an orange solid (138 mg, 0.217mmol, 53% yield based on 5,5'-bis(tributylstannyl)-2,2'-bithiophene). The chemical reaction formula is shown below.
[Chem. 28]

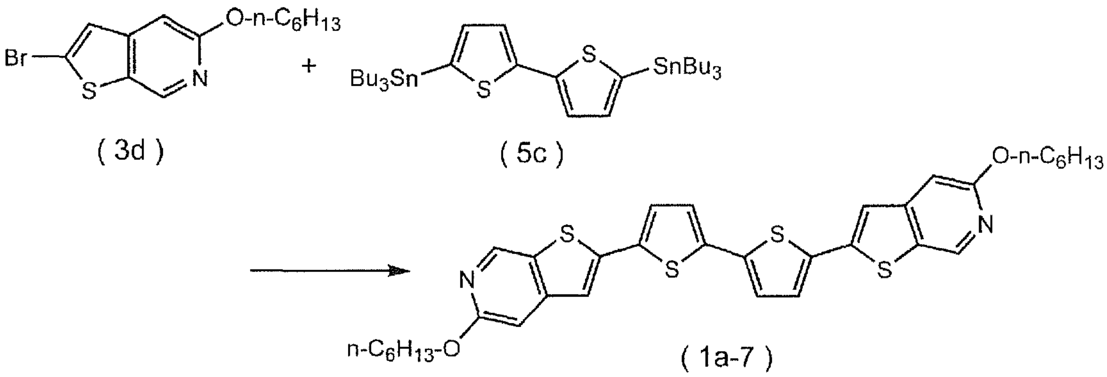

( 3d ) ( 5c ) ( 1a-7 )

The NMR data of 5,5'-bis(5-n-hexyloxythieno[2,3-c]pyridin-2-yl)-2,2'-bithio phene represented by the formula (1a-7) were as follows. $^1$H-NMR Spectrum (400MHz, $CDCl_3$) : δ0.99 (t, J=7.2Hz, 6H), 1.33-1.37 (m, 8H), 1.45-1.51 (m, 4H), 1.79-1.86 (m, 4H), 4.35 (t, J=6.5Hz, 4H), 7.26 (d, J=3.9Hz, 2H), 7.28 (d, J=3.9Hz, 2H), 7.45 (s, 2H), 7.73 (s, 2H), 8.73 (s, 2H).

Example 4

(Synthesis Example 7) [Synthesis of

2,5-bis(5-phenylsulfonythieno[2,3-c]pyridin-2-yl)thiophene represented by formula (1a-8)]

A reaction and purification were carried out in the same manner as Synthesis Example 5-1 except that 2-bromo-5-methoxythieno[2,3-c]pyridine (200 mg, 0.82 mmol) was changed to 2-bromo-5-phenylsulfonylthieno[2,3-c]pyridine (290 mg, 0.82 mmol) obtained by Synthesis Example 2 and 2,5-bis(tributylstannyl)thiophene (271 mg, 0.41 mmol) was changed to 2,5-bis(trimethylstannyl)thiophene (168 mg, 0.41mmol), so that a target compound, i.e., 2,5-bis(5-phenylsulfonythieno[2,3-c]pyridin-2-yl)thiophene, was obtained as an orange solid (150 mg, 0.24 mmol, 58% yield based on 2,5-bis(trimethylstannyl)thiophene). The chemical reaction formula is shown below.

[Chem. 29]

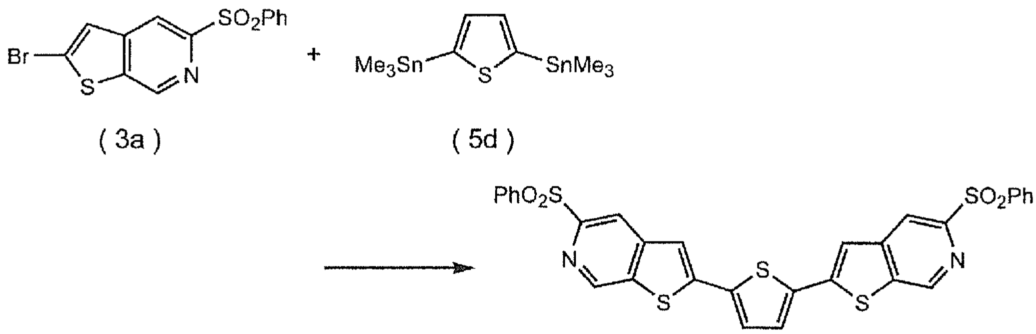

( 3a ) ( 5d ) ( 1a-8 )

The NMR data of 2,5-bis(5-phenylsulfonythieno[2,3-c]pyridin-2-yl)thiophene represented by the formula (1a-8) were as follows. $^1$H-NMR Spectrum (270MHz, $CDCl_3$, TMS, ppm) δ: 9.13 (d, 2H, J=1.0Hz), 8.88 (d, 2H, 1.0Hz), 8.10 (dd, 4H, J=1.1Hz), 7.81 (s, 2H), 7.61-7.46 (m, 6H), 7.39 (d, 2H, J=3.51Hz).

Example 5

(Synthesis Example 8) [Synthesis of 5,5'-bis(5-phenylsulfonythieno[2,3-c]pyridin-2-yl)-2,2'-bit hiophene represented by formula (1a-9)]

A reaction and purification were carried out in the same manner as Synthesis Example 5-1 except that 2-bromo-5-methoxythieno[2,3-c]pyridine (200 mg, 0.82 mmol) was changed to 2-bromo-5-phenylsulfonylthieno[2,3-c]pyridine (290 mg, 0.82 mmol) obtained by Synthesis Example 2 and 2,5-bis(tributylstannyl)thiophene (271 mg, 0.41 mmol) was changed to 5,5'-bis(trimethylstannyl)-2,2'-bithiophene (202 mg, 0.41mmol), so that a target compound, i.e., 5,5'-bis(5-phenylsulfonythieno[2,3-c]pyridin-2-yl)-2,2'-bit hiophene, was obtained as an orange solid (140 mg, 0.20 mmol, 48% yield based on 2,5-bis(tributylstannyl)thiophene). The chemical reaction formula is shown below.

[Chem. 30]

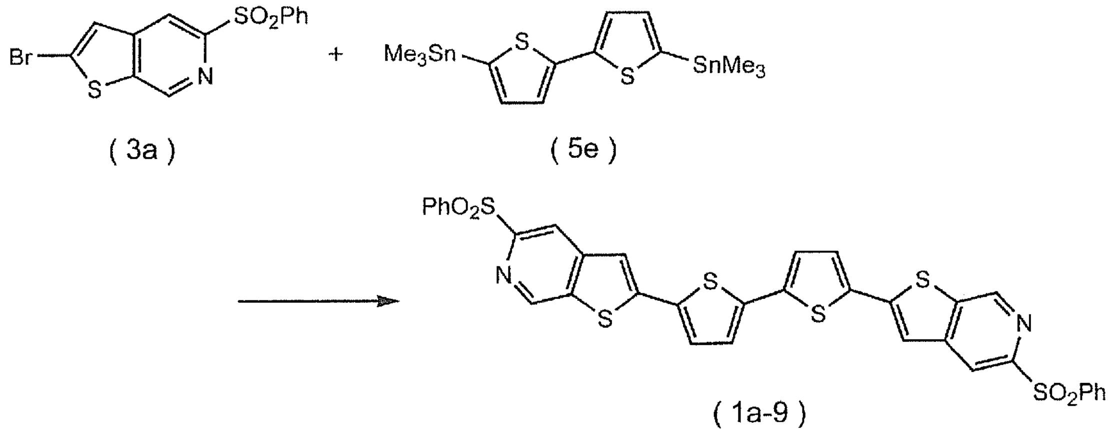

( 3a ) ( 5e )

( 1a-9 )

The NMR data of 5,5'-bis(5-phenylsulfonythieno[2,3-c]pyridin-2-yl)-2,2'-bit hiophene represented by the formula (1a-9) were as follows. $^1$H-NMR Spectrum (270MHz, $CDCl_3$, TMS, ppm) δ: 9.17 (d, 2H, J=1.08Hz), 8.91 (d, 2H, 1.08Hz), 8.11 (dd, 4H, J=1.08Hz), 7.89 (s, 2H), 7.61-7.48 (m, 6H), 7.30 (d, 2H, J=3.51Hz), 7.07 (dd, 2H, J=3.51Hz, 5.13Hz).

Example 6

(Synthesis Example 9) [Synthesis of

5-butoxycarbonylthieno[2,3-c]pyridine represented by formula (6b)]

Operations were done in the same manner as Synthesis Example 1 except that n-butyl cyanoformate (2.87 g, 22.6 mmol) was used instead of phenylsulfonyl cyanide (3.77 g, 22.6 mmol), so that 3.00 g of 5-butoxycarbonylthieno [2, 3-c] pyridine (12.8 mmol, 85% yield) was obtained. The chemical reaction formula is shown below.

[Chem. 31]

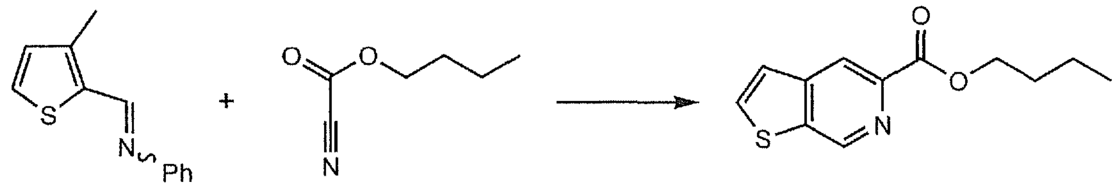

( 6b )

The NMR data of 5-butoxycarbonylthieno[2,3-c]pyridine represented by the formula (6b) were as follows. $^1$H-NMR Spectrum (270MHz, $CDCl_3$, TMS, ppm) δ: 9.26 (d, 1H, J=1.08Hz), 8.58 (d, 1H, J=1.08Hz), 7.82 (d, 1H, J=5.4Hz), 7.50 (d, 1H, J=5.4Hz), 4.46 (t, 2H, J=7.02Hz), 1.90-1.79 (m, 2H), 1.55-1.43 (m, 2H), 0.99 (t, 3H, J=7.56Hz).

(Synthesis Example 10) [Synthesis of 2-bromo-5-butoxycarbonylthieno[2,3-c]pyridine represented by formula (3e)]

Operations were carried out in the same manner as Synthesis Example 2 except that 5-butoxycarbonylthieno[2,3-c]pyridine (2.02 g, 8.6 mmol) was used instead of 5-phenylsulfonylthieno [2, 3-c]pyridine (2. 37 g, 8.6 mmol), so that 2.43 g of 2-bromo-5-butoxycarbonylthieno[2,3-c]pyridine (7.7 mmol, 90% yield) was obtained. The chemical reaction formula is shown below.

[Chem. 32]

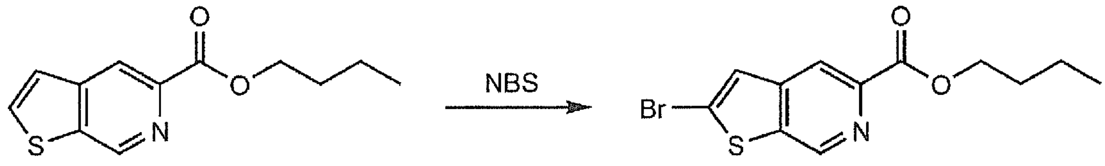


( 6b ) ( 3e )

The NMR data of 2-bromo-5-butoxycarbonylthieno [2, 3-c]pyridine represented by the formula (3e) were as follows. $^1$H-NMR Spectrum (270MHz, $CDCl_3$, TMS, ppm) δ: 9.24 (d, 1H, J=1.08Hz), 8.57 (d, 1H, J=1.08Hz), 7.81 (d, 1H, J=4.05Hz), 4.49 (t, 2H, J=6.48Hz), 1.91-1.78 (m, 2H), 1.57-1.44 (m, 2H), 0.97 (t, 3H, J=7.29Hz).

(Synthesis Example 11) [Synthesis of (2-(2,2':5',2"-thiophen-2-yl)-5-butoxycarbonylthieno[2,3-c] pyridine) represented by formula (1c)]

To a three-necked, 50-ml flask equipped with a thermometer and a magnetic stirrer, 2-bromo-5-butoxycarbonylthieno[2,3-c]pyridine (1.57 g, 5.0 mmol), 2-(tributylstannyl) -2,2':5',2"-thiophene (3.22 g, 6. 0 mmol), tetrakistriphenylphosphino palladium (57.8 mg, 0.05 mmol), and 15 ml of tetrahydrofuran were added, and after the replacement of the atmosphere in the system with argon, heating was done at 60°C for seven hours under stirring. After the completion of the reaction, the reaction solution was added to 100 ml of water and then the organic phase was separated with a separatory funnel. The aqueous phase was extracted with methylene chloride, whose mixture with the previously mentioned organic phase was washed with water, and then the organic phase was dried with anhydrous magnesium sulfate. The residue obtained by concentrating the organic phase under reduced pressure was purified by silica gel column chromatography (developing solvent: hexane/ethyl acetate = 80/20), obtaining a target compound, i.e., 2-(2,2':5',2"-thiophen-2-yl)-5-butoxycarbonylthieno[2,3-c]p yridine, as an orange solid (1.93 g, 4.0 mmol, 80% yield based on 2-bromo-5-butoxycarbonylthieno[2,3-c]pyridine). The chemical reaction formula is shown below.

[Chem. 33]

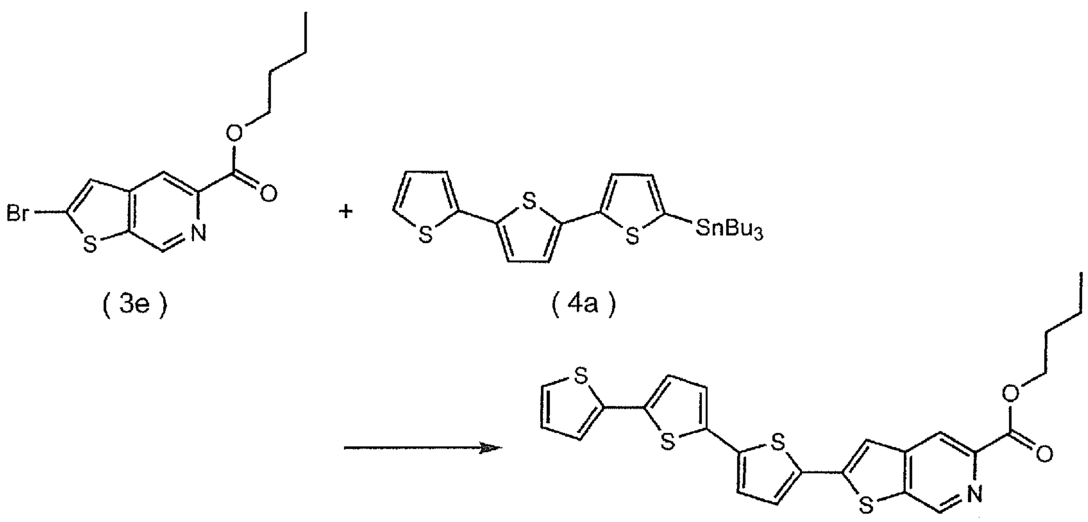

( 3e ) ( 4a )

( 1c )

The NMR data of (2-(2,2':5',2"-thiophen-2-yl)-5-butoxycarbonylthieno[2,3-c] pyridine) represented by the formula (1c) were as follows. $^1$H-NMR Spectrum (270MHz, $CDCl_3$, TMS, ppm) δ: 9.29 (d, 1H, J=1.08Hz), 8.88 (d, 2H, 1.08Hz), 7.85 (s, 1H), 7.33 (d,

1H, J=3.51Hz), 7.28-7.20 (m, 2H), 7.16 (d, 1H, J=3.51Hz), 7.12 (d, 1H, J=3.51Hz), 7.05 (dd, 1H, J=3.51Hz, 4.86Hz), 4.49 (t, 3H, J=6.48Hz), 1.91-1.81 (m, 2H), 1.57-1.47 (m, 2H), 1.00 (t, 3H, J=7.29Hz).

Example 7

[Preparation of organic thin film transistor element, and measurement of carrier mobility]

A field effect transistor (organic thin film transistor) of a bottom contact type structure illustrated in Fig. 1 was prepared by the following procedure. As illustrated in Fig. 1, the field effect transistor prepared in this example is one using a doped silicon substrate 8 that serves as both a substrate and a gate electrode, and 7 depicted in Fig. 1 is a contact (gate contact).

The doped silicon wafer (substrate (gate electrode) 8) was coated with a 230-nm thick $SiO_2$ film (insulator layer 2) by thermal oxidation. The rear side (the surface of the substrate 8 opposite to the side of the insulator layer 2) was etched with an aqueous hydrofluoric acid solution, thereby removing the $SiO_2$ film having been formed on the rear side, and then gold was vapor deposited on the bare substrate 8 by the electron beam method, whereby a gate contact 7 was produced. Then, onto the insulator layer 2 formed on the substrate 8, gold was vapor deposited by the electron beam method, whereby a source electrode 3 and a drain electrode 4 were formed. The channel length was 10 μm and the channel width was 2 cm. Additionally, a semiconductor layer 1 was formed by vacuum vapor deposition from the respective compounds obtained in Example 1 to 6, whereby field effect transistors of the present invention were prepared.

The characteristics of the thus-prepared field effect transistors were measured at room temperature under vacuum by using a voltage/current generator (R6246, manufactured by ADVANTEST Corporation). The FET property of an element was measured at room temperature under vacuum. The measurement was carried out by applying a voltage of from 0 to -100 V to between the source and drain electrodes of the field effect transistor prepared, varying the voltage applied to between the source and gate electrodes within the range of 0 V to -100 V, and measuring a current that flows for the voltage ($V_d$) applied to between the source and drain electrodes. From the measurement data, a carrier mobility (μ) was calculated using the formula

$$i_d = \{W_\mu (V_g - V_t)^2 C_i\}/2L.$$

In the formula, $i_d$ represents a current that flows for the voltage $V_d$ applied to between the source and drain electrodes, $V_g$ represents a voltage applied to between the source and gate electrodes, $V_t$ represents a threshold voltage, $C_i$ represents the electrostatic capacitance per unit area of the insulator layer, W represents the channel width, and L represents the channel length. An on off ratio was calculated as a ratio of the maximum to the minimum of the current flowed between the source and drain electrodes when a voltage of -100 V was applied to between the source and drain electrodes and the voltage applied to between the source and gate electrodes was varied within the range of from 0 V to -100 V. In the following Table 1, the carrier mobilities (μ) and the on off ratios of field effect transistors are shown.

[Table 1]

| Compound | Carrier mobility (μ) ($cm^2/Vs$) | On off ratio |
|---|---|---|
| 1a-1 | $2.1\times10^{-3}$ | $10^4$ |
| 1a-5 | $8.9\times10^{-4}$ | $10^5$ |
| 1a-8 | $1.2\times10^{-3}$ | $10^5$ |
| 1a-9 | $3.9\times10^{-3}$ | $10^4$ |
| 1b-1 | $4.3\times10^{-3}$ | $10^5$ |
| 1c | $5.1\times10^{-4}$ | $10^5$ |

Explanation of symbols

1 Semiconductor layer

2 Insulator layer

3 Source electrode

4 Drain electrode

7 Gate contact

8 Substrate (gate electrode)

## Claims

1. A thienopyridine derivative represented by the following general formula (1):

[Chem. 1]

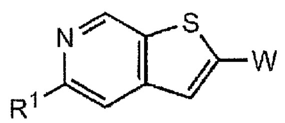

(1)

wherein $R^1$ is a hydrogen atom, an alkyl group that may have a substituent, an alkenyl group that may have a substituent, an alkynyl group that may have a substituent, an alkoxy group that may have a substituent, an aryl group that may have a substituent, a heteroaromatic ring group that may have a substituent, an alkylthio group that may have a substituent, an arylthio group that may have a substituent, an ester group that may have a substituent, or a substituent represented by $-SO_2R^2$ (wherein $R^2$ is a hydrocarbon group having 1 to 20 carbon atoms that may have a substituent), W is at least one member selected from the group consisting of aryl groups that may have a substituent and heteroaromatic ring groups that may have a substituent.

2. The thienopyridine derivative according to claim 1, wherein W is a heteroaromatic ring group represented by the following general formula (2):

[Chem. 2]

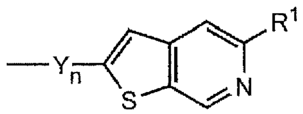

(2)

wherein $R^1$ is the same as defined above for the general formula (1), Y is at least one member selected from the group consisting of arylene groups and divalent heteroaromatic ring groups, and n is an integer of not less than 0.

3. A method for producing the thienopyridine derivative according to claim 1 or 2, wherein the method comprises subjecting a compound represented by the following general formula (3):

[Chem. 3]

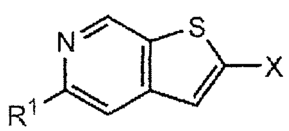

(3)

wherein $R^1$ is the same as defined above for the general formula (1) and X is a halogen atom, and a compound represented by the following general formula (4):

[Chem. 4] W-Z (4)

wherein W is the same as defined above, Z is one member selected from the group consisting of -MgCl, -MgBr, -MgI, -ZnCl, -ZnBr, -ZnI, $-Sn(R^3)_3$ (wherein $R^3$ is an alkyl group having 1 to 10 carbon atoms or aryl group that may have a substituent), $-Si(OH)_3$, a boronic acid group, and a boronic acid ester group to a cross-coupling reaction.

**4.** A method for producing the thienopyridine derivative according to claim 1 or 2, wherein the method comprises subjecting a compound represented by the following general formula (3):

[Chem. 5]

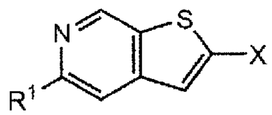

(3)

wherein $R^1$ is the same as defined above for the general formula (1) and X is a halogen atom, and a compound represented by the following general formula (5):

[Chem. 6]

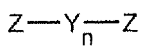

(5)

wherein Y is at least one member selected from the group consisting of arylene groups and divalent heteroaromatic ring groups, n is an integer of not less than 1, Z is one member selected from the group consisting of -MgCl, -MgBr, -MgI, -ZnCl, -ZnBr, -ZnI, $-Sn(R^3)_3$ (wherein $R^3$ is an alkyl group having 1 to 10 carbon atoms or aryl group that may have a substituent), $-Si(OH)_3$, a boronic acid group, and a boronic acid ester group to a cross-coupling reaction.

**5.** A method for producing the thienopyridine derivative according to claim 1 or 2, wherein a compound represented by the following general formula (3):

[Chem. 7]

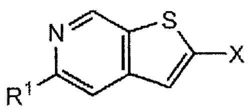

(3)

wherein $R^1$ is the same as defined above for the general formula (1) and X is a halogen atom is lithiated with an organolithium compound and subsequently copper (II) halide is caused to react therewith.

**6.** The method for producing the thienopyridine derivative according to any one of claims 3 to 5, wherein a compound represented by the following general formula (6):

[Chem. 8]

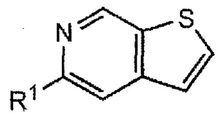

(6)

wherein $R^1$ is the same as defined above for the general formula (1) is caused to react with a halogenating agent to obtain a compound represented by the following general formula (3):

[Chem. 9]

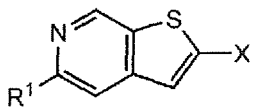

(3)

wherein $R^1$ is the same as defined above for the general formula (1) and X is a halogen atom.

**7.** A compound represented by the following general formula (3):

[Chem. 10]

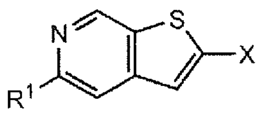

(3)

wherein $R^1$ is a hydrogen atom, an alkyl group that may have a substituent, an alkenyl group that may have a substituent, an alkynyl group that may have a substituent, an alkoxy group that may have a substituent, an aryl group that may have a substituent, a heteroaromatic ring group that may have a substituent, an alkylthio group that may have a substituent, an arylthio group that may have a substituent, an ester group that may have a substituent, or a substituent represented by $-SO_2R^2$ (wherein $R^2$ is a hydrocarbon group having 1 to 20 carbon atoms that may have a substituent), and X is a halogen atom.

**8.** An organic semiconductor device comprising the thienopyridine derivative according to claim 1 or 2.

**9.** The organic semiconductor device according to claim 8, wherein the organic semiconductor device is an organic thin film transistor element.

Figure 1

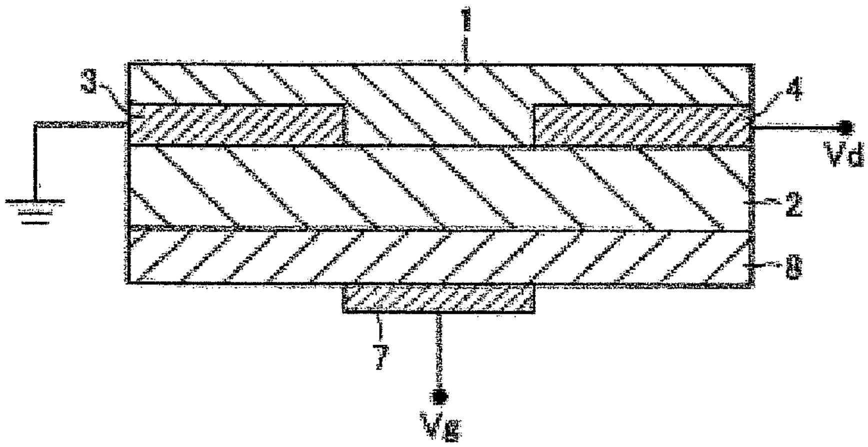

**INTERNATIONAL SEARCH REPORT**

International application No.
PCT/JP2011/055963

A. CLASSIFICATION OF SUBJECT MATTER
*C07D495/04*(2006.01)i, *H01L29/786*(2006.01)i, *H01L51/05*(2006.01)i, *H01L51/30*(2006.01)i

According to International Patent Classification (IPC) or to both national classification and IPC

B. FIELDS SEARCHED

Minimum documentation searched (classification system followed by classification symbols)
C07D495/04, H01L29/786, H01L51/05, H01L51/30

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X/Y | GRAULICH, A. et al, A rapid synthesis of thieno[2,3-c]pyridine and 2-substituted thieno[2,3-c]pyridines, Synthesis, 2004, No.12, pp.1935-1937 | 1,7/3,4,6,7 |
| X/Y | WO 2008/047198 A1 (Graceway Pharmaceuticals, L.L.C.), 24 April 2008 (24.04.2008), entire text; particularly, claims; examples 11, 12 & US 2008/090861 A1 & EP 2074128 A1 & JP 2010-506895 A | 1/3,4,6,7 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 05 April, 2011 (05.04.11) | 12 April, 2011 (12.04.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No.
PCT/JP2011/055963

C (Continuation). DOCUMENTS CONSIDERED TO BE RELEVANT

| Category* | Citation of document, with indication, where appropriate, of the relevant passages | Relevant to claim No. |
|---|---|---|
| X/Y | WO 2007/084391 A2 (AMGEN INC.), 26 July 2007 (26.07.2007), entire text; particularly, scheme; example 108 & US 2007/173506 A1 & EP 1981884 A2 & US 7514566 B2 & JP 2009-525960 A | 1/3,4,6,7 |
| X/Y | JP 2006-316054 A (TANABE SEIYAKU CO.), 24 November 2006 (24.11.2006), entire text; particularly, methods 2, 6; compound no.23 (Family: none) | 1/3,4,6,7 |
| X/Y | NERENZ, H. et al, Nonlinear optical chromophores with isoquinolines, thieno[2, 3-c]pyridines and 2-(2'-thienyl)pyridines as inherently polarized π-electron bridges, Journal of the Chemical Society, Perkin Transactions 2: Physical Organic Chemistry, 1998, No.2, pp.437-448 | 1/3,4,6,7 |
| X/Y | WO 2006/067532 A1 (PROSIDION LTD., GB), 29 June 2006 (29.06.2006), entire text; particularly, examples 37, 41, 42 & EP 1838706 A1 & JP 2008-525417 A & US 2009/099227 A1 | 1/3,4,6,7 |
| X/Y | GLEICH, E. et al, Elemental sulfur reactions with 4-picoline, Phosphorus and Sulfur and the Related Elements, 1986, Vol.28, No.3, pp.315-25 | 1/3,4,6,7 |
| A | WO 2009/139339 A1 (TORAY IND. INC., JP), 19 November 2009 (19.11.2009), entire text; particularly, claims (Family: none) | 1-9 |
| A | WO 2009/103706 A1 (UNIV DENMARK TECH, DK), 27 August 2009 (27.08.2009), entire text; particularly, claims; examples (Family: none) | 1-9 |
| A | JP 2004-31004 A (KONICA CORP.), 29 January 2004 (29.01.2004), entire text; particularly, claims; examples (Family: none) | 1-9 |
| A | JP 2001-89474 A (FUJI PHOTO FILM CO., LTD.), 03 April 2001 (03.04.2001), entire text; particularly, claims; examples (Family: none) | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **T. OTSUBO et al.** Synthesis, Optical, and Conductive Properties of Long Oligothiophenes and Their Utilization as Molecular Wires. *Bull. Chem. Soc. Jpn.,* 2001, vol. 74 (10), 1789-1801